# EUROPEAN PATENT SPECIFICATION

(11) **EP 3 470 490 B1**
(45) Date of publication and mention of the grant of the patent: **11.11.2026**
(21) Application number: 18199159.7
(22) Date of filing: 18.08.2009
(51) Int. Cl.: C09K 5/04, C09K 3/30, F25B 9/00, F25B 30/00

(54) **USE OF AZEOTROPE-LIKE COMPOSITIONS OF 1,1,1,2-TETRAFLUOROPROPENE AND 1,1,1,2-TETRAFLUOROETHANE**
VERWENDUNG VON AZEOTROPARTIGE ZUSAMMENSETZUNGEN AUS 1,1,1,2-TETRAFLUORPROPEN UND 1,1,1,2-TETRAFLUORETHAN
UTILISATIONS DES COMPOSITIONS DE TYPE AZÉOTROPE DE 1,1,1,2-TÉTRAFLUOROPROPÈNE ET DE 1,1,1,2-TÉTRAFLUOROÉTHANE

(30) Priority: 19.08.2008 US 89986 P; 17.08.2009 US 542185
(43) Date of publication of application: 17.04.2019
(62) Document number(s) of the earlier application(s) in accordance with Art. 76 EPC: 09808686.1 / 2 313 471
(73) Proprietor: Solstice Advanced Materials US, Inc., Morris Plains, NJ 07950 (US)
(72) Inventor: SEETON, Christopher, J., Morris Plains, NJ 07950 (US); PHAM, Hang, T., Amherst, NY New York 14228 (US); SINGH, Rajiv, R., Getzville, NY New York 14068 (US); HULSE, Ryan, Getzville, NY New York 14068 (US); SPATZ, Mark, W., East Amherst, NY New York 14051 (US); WILSON, David, P., East Amherst, NY New York 14051 (US); YANA MOTTA, Samuel, F., East Amherst, NY New York 14051 (US)
(74) Representative: Stepney, Gregory John

(56) References cited:
- WO-A1-2010/002020
- US-A1- 2005 245 421
- US-A1- 2006 243 945
- US-A1- 2007 007 488

## Description

### BACKGROUND OF THE INVENTION

### Field Of The Invention

The present invention provides azeotrope-like compositions of 1,1,1,2-tetrafluoropropene (HFO-1234yf) and 1,1,1,2-tetrafluoroethane (HFC-134a), and uses thereof, including use in refrigerant compositions and refrigeration systems.

### Description of the Related Art

Fluorocarbon based fluids have found widespread use in industry in a number of applications, including as refrigerants, aerosol propellants, blowing agents, heat transfer media, and gaseous dielectrics. Because of the suspected environmental problems associated with the use of some of these fluids, including the relatively high global warming potentials associated therewith, it is desirable to use fluids having low or even zero ozone depletion potential, such as hydrofluorocarbons ("HFCs"), and low global warming potential. Thus, the use of fluids that do not contain chlorofluorocarbons ("CFCs") or hydrochlorofluorocarbons ("HCFCs") is desirable. The use of alkenes is also desirable due to there short atmospheric lifetime which results in a relatively low global warming potential. Additionally, the use of single component fluids or azeotropic mixtures, which do not fractionate on boiling and evaporation, is desirable. However, the identification of new, environmentally-safe, non-fractionating mixtures is complicated due to the fact that azeotrope formation is not readily predictable. The industry is continually seeking new fluorocarbon based mixtures that offer alternatives, and are considered environmentally safer substitutes for CFCs and HCFCs. Of particular interest are mixtures containing hydrofluorocarbons, hydrofluorolefins ("HFOs") and other fluorinated compounds, which have a zero ozone depletion potentials and low global warming potential. Such mixtures are the subject of this invention. US 2006/243945 A1, US 2007/007488 A1, WO 2010/002020 A1 and US 2005/245421 A1 describe compositions containing fluorine substituted olefins.

Described herein are compositions that help to satisfy the continuing need for alternatives to CFCs and HCFCs. Described herein are azeotrope-like compositions comprising of 1,1,1,2-tetrafluoropropene (HFO-1234yf) and 1,1,1,2-tetrafluoroethane (HFC-134a). The preferred compositions described herein tend to exhibit relatively low global warming potentials ("GWPs"). Accordingly, such compositions can be used to great advantage in a number of applications, including as replacements for CFCs, HCFCs, and HFCs (such as HFC-134a) in refrigerant, aerosol, blowing agents, and other applications. This azeotrope-like composition can be used as a replacement in systems already utilizing HFC-134a where a significant reduction in GWP is desired. Additionally, it has been surprisingly found that azeotrope-like compositions of HFO-1234yf and HFC-134a can be formed. Accordingly, the described herein are methods of producing an azeotrope-like composition comprising combining HFO-1234yf and HFC-134a in amounts effective to produce an azeotrope-like composition. In addition, it has been found that the azeotrope-like compositions described herein exhibit properties that make that make them advantageous for use as, or in, refrigerant, aerosol, and blowing agent compositions. Accordingly, described herein are refrigerant compositions comprising an azeotrope-like composition of HFO-1234yf and HFC-134a.

### SUMMARY OF THE INVENTION

The invention provides use of an azeotrope-like composition consisting essentially of effective amounts of 1,1,1,2-tetrafluoropropene and 1,1,1,2-tetrafluoroethane as set out in the appended claims.

Also described is a method of recharging a refrigerant system that contains a refrigerant to be replaced and a lubricant comprising the steps of: (a) removing the refrigerant to be replaced from the refrigeration system while retaining a substantial portion of the lubricant in said system; and (b) introducing to the system a refrigerant composition comprising the above azeotrope-like composition.

### DETAILED DESCRIPTION OF THE INVENTION

Described herein are azeotrope-like compositions comprising 1,1,1,2-tetrafluoropropene (HFO-1234yf) and 1,1,1,2-tetrafluoroethane (HFC-134a), and uses thereof, including use in refrigerant compositions and refrigeration systems.

As used herein, the term "azeotrope-like" is intended in its broad sense to include both compositions that are strictly azeotropic and compositions that behave like azeotropic mixtures. From fundamental principles, the thermodynamic state of a fluid is defined by pressure, temperature, liquid composition, and vapor composition. An azeotropic mixture is a system of two or more components in which the liquid composition and vapor composition are equal at the stated pressure and temperature. In practice, this means that the components of an azeotropic mixture are constant-boiling and cannot be separated during a phase change. The azeotrope-like compositions described herein may include additional components that do not form new azeotrope-like systems, or additional components that are not in the first distillation cut. The first distillation cut is the first cut taken after the distillation column displays steady state operation under total reflux conditions. One way to determine whether the addition of a component forms a new azeotrope-like system so as to be outside of this invention is to distill a sample of the composition with the component under conditions that would be expected to separate a non-azeotropic mixture into its separate components. If the mixture containing the additional component is non-azeotrope-like, the additional component will fractionate from the azeotrope-like components. If the mixture is azeotrope-like, some finite amount of a first distillation cut will be obtained that contains all of the mixture components that is constant boiling or behaves as a single substance. It follows from this that another characteristic of azeotrope-like compositions is that there is a range of compositions containing the same components in varying proportions that are azeotrope-like or constant boiling. All such compositions are intended to be covered by the terms "azeotrope-like" and "constant boiling". As an example, it is well known that at differing pressures, the composition of a given azeotrope will vary at least slightly, as does the boiling point of the composition. Thus, an azeotrope of A and B represents a unique type of relationship, but with a variable composition depending on temperature and/or pressure. It follows that, for azeotrope-like compositions, there is a range of compositions containing the same components in varying proportions that are azeotrope-like. All such compositions are intended to be covered by the term azeotrope-like as used herein. It is well-recognized in the art that it is not possible to predict the formation of azeotropes. (See, for example, U.S. Patent No. 5,648,017 (column 3, lines 64-65) and U.S. Patent No. 5,182,040 (column 3, lines 62-63)). It has been unexpectedly discovered that HFO-1234yf and HFC-134a form azeotrope-like compositions.

According to certain preferred embodiments, the azeotrope-like compositions described herein comprise, and preferably consist essentially of, effective amounts of HFO-1234yf and HFC-134a. The term "effective amounts" as used herein refers to the amount of each component which upon combination with the other component, results in the formation of an azeotrope-like composition of the present invention. The azeotrope-like compositions described herein can be produced by combining effective amounts of HFO-1234yf and HFC-134a. Any of a wide variety of methods known in the art for combining two or more components to form a composition can be adapted for use in the present methods to produce an azeotrope-like composition. For example, HFO-1234yf and HFC-134a can be mixed, blended, or otherwise contacted by hand and/or by machine, as part of a batch or continuous reaction and/or process, or via combinations of two or more such steps. In light of the disclosure herein, those of skill in the art will be readily able to prepare azeotrope-like compositions described herein without undue experimentation.

The HFO-1234yf and HFC-134a are present in amounts effective to produce an azeotrope-like composition. The HFO-1234yf is present in the azeotrope-like composition in an amount of from 50 to 55 weight percent. The HFC-134a, is present in the azeotrope-like composition in an amount of from 45 to 50 weight percent. Usually the inventive azeotrope-like compositions have a boiling point of from about -30.0°C to about -29.0°C at a pressure of about 14.3 psia.

The azeotrope-like compositions of this invention may also be used as refrigerant compositions. The refrigerant compositions of the present invention may be used in any of a wide variety of refrigeration systems including air-conditioning, refrigeration, heat-pump systems, and the like. In certain preferred embodiments, the compositions of the present invention are used in refrigeration systems originally designed for use with an HFC refrigerant, such as, for example, HFC-134a. The preferred compositions of the present invention tend to exhibit many of the desirable characteristics of HFC-134a and other HFC-refrigerants, including non-flammability or low flammability, and a GWP that is as low, or lower than that of conventional HFC-refrigerants. In addition, the relatively constant boiling nature of the compositions of the present invention makes them even more desirable than certain conventional HFCs for use as refrigerants in many applications.

In certain embodiments, the compositions of the present invention may be used to retrofit refrigeration systems containing a refrigerant which comprises a chlorofluorocarbon, a hydrofluorocarbon, a hydrochlorofluorocarbon, a chlorofluorcarbon, or combinations thereof and lubricants conventionally used therewith. Such as a mineral oil, alkybenezene (AB), polyalphaolefin (PAO), polyol ester (POE), polyakylene glycol (PAG), polyvinyl ether (PVE), synthetic naphthalene, fluorolubricant or combinations thereof. The method comprises removing or leaking at least a portion of the refrigerant from the refrigeration apparatus and leaving a residue comprising the lubricant, and adding to said residue the inventive azeotrope-like composition. Preferred refrigeration compositions of the present invention may be used in refrigeration systems containing a lubricant used conventionally with CFC HCFC, and/or CFC-refrigerants, such as mineral oils, silicone oils, and the like, or may be used with other lubricants traditionally used with such refrigerants. Preferably, the present methods involve recharging a refrigerant system that contains a refrigerant to be replaced and a lubricant comprising the steps of (a) removing the refrigerant to be replaced from the refrigeration system while retaining a substantial portion of the lubricant in said system; and (b) introducing to the system a composition of the present invention. As used herein, the term "substantial portion" refers generally to a quantity of lubricant which is at least about 50% (by weight) of the quantity of lubricant contained in the refrigeration system prior to removal of the refrigerant. Preferably, the substantial portion of lubricant in the system according to the present invention is a quantity of at least about 60% of the lubricant contained originally in the refrigeration system, and more preferably a quantity of at least about 70%. As used herein the term "refrigeration system" refers generally to any system or apparatus, or any part or portion of such a system or apparatus, which employs a refrigerant to provide cooling to one space thereby heating another. Such refrigeration systems include, for example, air conditioners including automotive air conditioning, electric refrigerators, chillers, transport refrigeration systems, commercial refrigeration systems and the like. Refrigeration systems may also include heat pump systems whereas the desired benefit is the heating provided by condensing the refrigerant stream and the cooling portion or evaporator extracts heat from the surroundings or other energy streams for use in such heating.

Any of a wide range of known methods can be used to remove refrigerants to be replaced from a refrigeration system while removing less than a major portion of the lubricant contained in the system. For example, because refrigerants are quite volatile relative to traditional hydrocarbon-based lubricants, such as mineral oil, alkybenezene (AB), polyalphaolefins (PAO), polyol esters (POE), polyakylene glycols (PAG), fluorolubricants and the like (the boiling points of refrigerants are generally less than 10°C whereas the boiling points of lubricants are generally more than 200 °C). In embodiments wherein the lubricant is a hydrocarbon-based lubricant, the removal step may readily be performed by pumping refrigerants in the gaseous state out of a refrigeration system containing liquid state lubricants. Such removal can be achieved in any of a number of ways known in the art, including, the use of a refrigerant recovery system, such as the recovery system manufactured by Robinair of Ohio. Alternatively, a cooled, evacuated refrigerant container can be attached to the low pressure side of a refrigeration system such that the gaseous refrigerant is drawn into the evacuated container and removed. Moreover, a compressor may be attached to a refrigeration system to pump the refrigerant from the system to an evacuated container. In light of the above disclosure, those of ordinary skill in the art will be readily able to remove the system refrigerant charge without removing the majority of the lubricant and charging with the disclosed azeotrope-like blend of HFC-134a and HFO-1234yf according to the present invention.

Another way for which refrigerant may be removed from the system is in the case of leaks, hose permeation or system failure in which an operator does not play an active part in the refrigerant removal, but rather the nature of the system seal, materials of construction, or operating conditions cause refrigerant removal. In this case if an operator "tops off a system where as the full refrigerant charge has not yet been removed. This is common practice in automotive air conditioning systems where the user experiences decreased performance and a service shops or the home user themselves recharge the system, pulling either HFO-1234yf into a system originally designed HFC-134a or pulling HFC-134a into a system originally designed for HFO-1234yf. This would constitute a partial charge, but would allow for azeotrope-like blend of HFC-134a and HFO-1234yf according to the present invention. Any of a wide range of methods for introducing the present refrigerant compositions to a refrigeration system can be used. For example, one method comprises attaching a refrigerant container to the low-pressure side of a refrigeration system and turning on the refrigeration system compressor to pull the refrigerant into the system. In such embodiments, the refrigerant container may be placed on a scale such that the amount of refrigerant composition entering the system can be monitored. When a desired amount of refrigerant composition has been introduced into the system, charging is stopped. Alternatively, a wide range of charging tools, known to those of skill in the art, is commercially available. Accordingly, in light of the above disclosure, those of skill in the art will be readily able to introduce the refrigerant compositions of the described herein into refrigeration systems described herein without undue experimentation.

Described herein are refrigeration systems comprising a refrigerant and methods of producing heating or cooling by condensing and/or evaporating a composition. In certain preferred embodiments, the methods for cooling an article comprise condensing a refrigerant composition comprising an azeotrope-like composition and thereafter evaporating said refrigerant composition in the vicinity of the article to be cooled. Certain preferred methods for heating an article comprise condensing a refrigerant composition comprising an azeotrope-like composition in the vicinity of the article to be heated and thereafter evaporating said refrigerant composition. In light of the disclosure herein, those of skill in the art will be readily able to heat and cool articles without undue experimentation.

### EXAMPLES

The following non-limiting examples serve to illustrate the invention.

### EXAMPLE 1

An ebulliometer consisting of vacuum jacketed tube with a condenser on top which is further equipped with a Quartz Thermometer is used. About 17.41 g of HFO-1234yf is charged to the ebulliometer and then HFC-134a is added in small, measured increments. Temperature depression is observed when HFC-134a is added to HFO-1234yf, indicating a binary minimum boiling azeotrope is formed. From greater than about 0 to about 60 weight percent HFC-134a, the boiling point of the composition stays below or around the boiling point of HFO-1234yf. The normal boiling temperature of HFC-134a is about -26.3 °C. The binary mixtures shown in Table 1 show the boiling point of the compositions did not go above the boiling point of HFO-1234yf. The compositions exhibit azeotrope and/or azeotrope-like properties over this range.

**TABLE 1**

| HFO-1234yf/HFC-134a Compositions at 98.6 kPaa (14.3 psia) | | |
|---|---|---|
| T (°C) | Wt.% HFO-1234yf | Wt.% HFC-134a |
| -28.97 | 100.0 | 0.00 |
| -29.13 | 97.81 | 2.19 |
| -29.63 | 89.83 | 10.17 |
| -29.66 | 86.27 | 13.73 |
| -29.86 | 75.89 | 24.11 |
| -29.91 | 71.29 | 28.71 |
| -29.92 | 65.16 | 34.84 |
| -29.91 | 60.68 | 39.32 |
| -29.81 | 56.27 | 43.73 |
| -29.67 | 52.71 | 47.29 |
| -29.38 | 47.10 | 52.90 |
| -29.16 | 43.38 | 56.62 |

### EXAMPLE 2

An azeotrope-like composition (50/50 by mass) of HFO-1234yf and HFC-134a was charged into an instrumented automotive air condition system and tested under the conditions typical for normal operation. The conditions are specified in a Society of Automotive Engineers (SAE) Standard J2765 as conditions experienced during operation of a motor vehicle. The system was evaluated at conditions representing full load cooling for ambient temperatures of 35, 45 and 50 °C as to exclude compressor cycling and system control influence of the results. Table 2 illustrates results from such testing. Lower discharge temperatures and higher suction pressures make this azeotrope-like blend attractive in refrigeration and heat pump operation. The lower discharge temperature adds robustness in thermal stability of system lubricants, materials and the refrigerant itself. The higher suction pressure competes against the higher discharge pressure of the mixtures; however, this allows for further heat exchanger optimization to achieve energy usage savings.

**TABLE 2**

| Results of MAC testing with azeotrope-like compositions of HFO-1234yf and HFC-134a | | | | | | | | |
|---|---|---|---|---|---|---|---|---|
| Condition* | | 145 | M45 | H45 | 150 | 135 | M35 | H35 |
| Discharge Temperature, °C | Blend | 79.8 | 90.47 | 103.4 | 88.4 | 74.6 | 87.9 | 101.1 |
| | HFC-134a Alone | 89.7 | 105.7 | 121.0 | 97.2 | 82.6 | 99.3 | 113.5 |
| | HFO-1234yf Alone | 80.2 | 93.0 | 105.4 | 87.6 | 73.8 | 88.4 | 101.5 |
| Discharge Pressure, MPa | Blend | 1.94 | 2.02 | 1.99 | 2.21 | 1.65 | 1.73 | 1.71 |
| | HFC-134a Alone | 1.91 | 1.92 | 1.90 | 2.20 | 1.60 | 1.63 | 1.63 |
| | HFO-1234yf Alone | 1.82 | 1.87 | 1.86 | 2.06 | 1.53 | 1.59 | 1.89 |
| Evaporation Temperature, °C | Blend | 9.4 | 2.4 | -0.2 | 13.1 | 10.5 | 2.6 | -0.1 |
| | HFC-134a Alone | 9.3 | 2.5 | 0.9 | 12.9 | 10.7 | 3.8 | 2.0 |
| | HFO-1234yf Alone | 10.0 | 3.9 | 2.2 | 13.0 | 10.8 | 4.5 | 3.0 |
| Suction Pressure, kPa | Blend | 432 | 316 | 275 | 487 | 446 | 314 | 272 |
| | HFC-134a Alone | 383 | 267 | 235 | 434 | 403 | 277 | 238 |
| | HFO-1234yf Alone | 404 | 294 | 258 | 446 | 411 | 293 | 253 |
| System Cooling Capacity, kW | Blend | 4.13 | 6.47 | 8.16 | 4.40 | 5.10 | 7.51 | 9.55 |
| | HFC-134a Alone | 4.26 | 6.67 | 8.22 | 4.51 | 5.12 | 7.97 | 9.71 |
| | HFO-1234yf Alone | 4.26 | 6.59 | 8.25 | 4.42 | 4.97 | 7.68 | 9.52 |
| System Efficiency, COP (kW/kW) | Blend | 2.1 | 1.3 | 1.1 | 2.0 | 2.9 | 1.7 | 1.4 |
| | HFC-134a Alone | 2.3 | 1.5 | 1.1 | 2.2 | 3.1 | 1.8 | 1.4 |
| | HFO-1234yf Alone | 2.2 | 1.4 | 1.1 | 2.1 | 3.0 | 1.7 | 1.3 |

| | | | | | | | | |
|---|---|---|---|---|---|---|---|---|
| * Condition are specified by SAE Standard J2765 wherein: 135 = automobile idling at 35 °C ambient temperature. 145 = automobile idling at 45 °C ambient temperature. 150 = automobile idling at 50 °C ambient temperature. M35 = automobile at medium speeds of 35-45 mph at 35 °C ambient temperature. M45 = automobile at medium speeds of 35-45 mph at 45 °C ambient temperature. H35 = automobile at high speeds of 65-80 mph at 35 °C ambient temperature. H45 = automobile at high speeds of 65-80 mph at 45 °C ambient temperature. | | | | | | | | |

The compositions are HFC-134a Alone, HFO-1234yf Alone and 50/50 by mass blends of HFC-134a and HFO-1234yf.

## Claims

1. The use of an azeotrope-like composition consisting essentially 50 to 55 weight % of 1,1,1,2-tetrafluoropropene (HFO-1234yf) and 45 to 50 weight % of 1,1,1,2-tetrafluoroethane (HFC-134a) as a replacement for a chlorofluorocarbon (CFC), a hydrochlorofluorocarbon (HCFC) and a hydrofluorocarbon (HFC) in a refrigeration system.

2. The use of a refrigerant composition comprising an azeotrope-like composition consisting essentially 50 to 55 weight % of 1,1,1,2-tetrafluoropropene (HFO-1234yf) and 45 to 50 weight % of 1,1,1,2-tetrafluoroethane (HFC-134a) as a replacement for a CFC, a HCFC and a HFC in a refrigeration system.

3. The use according to claim 2, in a refrigeration system including air-conditioning. Refrigeration and heat-pump systems.

4. The use according to claim 2, in a refrigeration system selected from the group consisting of air conditioners including automotive air conditioning, electric refrigerators, chillers, transport refrigeration systems and commercial refrigeration systems.

5. The use according to claim 1, wherein the HFC is HFC-134a.

6. The use according to claim 2, in a system originally designed for use with an HFC refrigerant, such as, for example, HFC-134a.

7. The use of an azeotrope-like composition consisting essentially 50 to 55 weight % of 1,1,1,2-tetrafluoropropene (HFO-1234yf) and 45 to 50 weight % of 1,1,1,2-tetrafluoroethane (HFC-134a) as a refrigerant composition in a system originally designed for use with an HFC refrigerant, such as, for example, HFC-134a.

8. The use according to claim 7, in a refrigeration system including air-conditioning, refrigeration and heat-pump systems.

9. The use according to claim 7, in a refrigeration system selected form the group consisting of air conditioners including automotive air conditioning, electric refrigerators, chillers, transport refrigeration systems and commercial refrigeration systems.

10. The use according to any one of claims 1 to 9, wherein the azeotrope-like composition consists essentially of 55 weight % of HFO-1234yf 45 weight % of HFC-134a.

11. The use according to any one of claims 1 to 10, wherein the azeotrope-like composition is provided in combination with a lubricant, preferably wherein the lubricant comprises mineral oil, alkylbenzene, polyalphaolefin, polyol ester, polyalkylene glycol. Polyvinyl ether, synthetic naphthalene, fluorolubricant or combinations thereof.

## Patentansprüche

1. Verwendung einer azeotropähnlichen Zusammensetzung, im Wesentlichen bestehend aus 50 bis 55 Gew.-% 1,1,1,2-Tetrafluorpropen (HFO-1234yf) und 45 bis 50 Gew.-% 1,1,1,2-Tetrafluorethan (HFC-134a) als Ersatz für einen Chlorfluorkohlenstoff (CFC), einen Hydrochlorfluorkohlenstoff (HCFC) und einen Hydrofluorkohlenstoff (HFC) in einem Kältesystem.

2. Verwendung einer Kältemittelzusammensetzung, umfassend eine azeotropähnliche Zusammensetzung, im Wesentlichen bestehend aus 50 bis 55 Gew.-% 1,1,1,2-Tetrafluorpropen (HFO-1234yf) und 45 bis 50 Gew.-% 1,1,1,2-Tetrafluorethan (HFC-134a) als Ersatz für einen CFC einen HCFC und einen HFC in einem Kältesystem.

3. Verwendung nach Anspruch 2 in einem Kältesystem, das Klimatisierungs-, Kälte- und Wärmepumpensysteme beinhaltet.

4. Verwendung nach Anspruch 2 in einem Kältesystem, das ausgewählt ist aus der Gruppe bestehend aus Klimaanlagen, die Automobilklimatisierung, elektrische Kältemaschinen, Kühler, Transportkältesysteme und kommerzielle Kältesysteme beinhalten.

5. Verwendung nach Anspruch 1, wobei das HFC HFC-134a ist.

6. Verwendung nach Anspruch 2 in einem System, das ursprünglich zur Verwendung mit einem HFC-Kältemittel, wie zum Beispiel HFC-134a, konzipiert wurde.

7. Verwendung einer azeotropähnlichen Zusammensetzung, im Wesentlichen bestehend aus 50 bis 55 Gew.-% 1,1,1,2-Tetrafluorpropen (HFO-1234yf) und 45 bis 50 Gew.-% 1,1,1,2-Tetrafluorethan (HFC-134a) als Kältemittelzusammensetzung in einem System, das ursprünglich zur Verwendung mit einem HFC-Kältemittel, wie zum Beispiel HFC-134a, konzipiert wurde.

8. Verwendung nach Anspruch 7 in einem Kältesystem, das Klimatisierungs-, Kälte- und Wärmepumpensysteme beinhaltet.

9. Verwendung nach Anspruch 7 in einem Kältesystem, das ausgewählt ist aus der Gruppe bestehend aus Klimaanlagen, die Automobilklimatisierung, elektrische Kältemaschinen, Kühler, Transportkältesysteme und kommerzielle Kältesysteme beinhalten.

10. Verwendung nach einem der Ansprüche 1 bis 9, wobei die azeotropähnliche Zusammensetzung im Wesentlichen aus 55 Gew.-% HFO-1234yf und 45 Gew.-% HFC-134a besteht.

11. Verwendung nach einem der Ansprüche 1 bis 10, wobei die azeotropähnliche Zusammensetzung in Kombination mit einem Schmierstoff bereitgestellt wird, wobei der Schmierstoff bevorzugt Mineralöl, Alkylbenzol, Polyalphaolefin, Polyolester, Polyalkylenglykol, Polyvinylether, synthetisches Naphthalin, Fluorschmierstoff oder Kombinationen davon umfasst.

## Revendications

1. Utilisation d'une composition de type azéotrope constituée essentiellement de 50 à 55 % en poids de 1,1,1,2-tétrafluoropropène (HFO-1234yf) et de 45 à 50 % en poids de 1,1,1,2-tétrafluoroéthane (HFC-134a) en tant que remplacement d'un chlorofluorocarbone (CFC), d'un hydrochlorofluorocarbone (HCFC) et d'un hydrofluorocarbone (HFC) dans un système de réfrigération.

2. Utilisation d'une composition réfrigérante comprenant une composition de type azéotrope constituée essentiellement de 50 à 55 % en poids de 1,1,1,2-tétrafluoropropène (HFO-1234yf) et de 45 à 50 % en poids de 1,1,1,2-tétrafluoroéthane (HFC-134a) en tant que remplacement d'un CFC, d'un HCFC et d'un HFC dans un système de réfrigération.

3. Utilisation selon la revendication 2, dans un système de réfrigération incluant des systèmes de climatisation, de réfrigération et de pompe à chaleur.

4. Utilisation selon la revendication 2, dans un système de réfrigération choisi dans le groupe constitué par des climatisations incluant des systèmes de climatisation d'automobile, de réfrigérateurs électriques, de refroidisseurs, de réfrigération de transport et des systèmes de réfrigération commerciaux.

5. Utilisation selon la revendication 1, dans laquelle le HFC est HFC-134a.

6. Utilisation selon la revendication 2, dans un système initialement destiné à être utilisé avec un réfrigérant HFC, tel que, par exemple, HFC-134a.

7. Utilisation d'une composition de type azéotrope constituée essentiellement de 50 à 55 % en poids de 1,1,1,2-tétrafluoropropène (HFO-1234yf) et de 45 à 50 % en poids de 1,1,1,2-tétrafluoroéthane (HFC-134a) en tant que composition réfrigérante dans un système initialement destiné à être utilisé avec un réfrigérant HFC, tel que, par exemple, HFC-134a.

8. Utilisation selon la revendication 7, dans un système de réfrigération incluant des systèmes de climatisation, de réfrigération et de pompe à chaleur.

9. Utilisation selon la revendication 7, dans un système de réfrigération choisi dans le groupe constitué par des climatisations incluant des systèmes de climatisation d'automobile, de réfrigérateurs électriques, de refroidisseurs, de réfrigération de transport et des systèmes de réfrigération commerciaux.

10. Utilisation selon l'une quelconque des revendications 1 à 9, dans laquelle la composition de type azéotrope est constituée essentiellement de 55 % en poids de HFO-1234yf et de 45 % en poids de HFC-134a.

11. Utilisation selon l'une quelconque des revendications 1 à 10, dans laquelle la composition de type azéotrope est fournie en combinaison avec un lubrifiant, de préférence dans laquelle le lubrifiant comprend une huile minérale, un alkylbenzène, une poly-alpha-oléfine, un ester de polyol, un polyalkylèneglycol, un polyvinyl-éther, un naphtalène synthétique, un lubrifiant fluoré, ou des combinaisons de ceux-ci.
